# EUROPÄISCHE PATENTSCHRIFT

(11) **EP 1 478 344 B1**
(45) Veröffentlichungstag und Bekanntmachung des Hinweises auf die Patenterteilung: **23.05.2007**
(21) Anmeldenummer: 03742925.5
(22) Anmeldetag: 30.01.2003
(51) Int. Cl.: A61K 9/16

(54) **SCHMELZEXTRUSION VON WIRKSTOFFSALZEN**
MELT EXTRUSION CONSISTING OF SALTS OF ACTIVE INGREDIENTS
EXTRUSION DE FUSION DE SELS D'AGENTS ACTIFS

(30) Priorität: 27.02.2002 DE 10208344
(43) Veröffentlichungstag der Anmeldung: 24.11.2004
(73) Patentinhaber: Röhm GmbH, 64293 Darmstadt (DE)
(72) Erfinder: PETEREIT, Hans-Ulrich, 64291 Darmstadt (DE); MEIER, Christian, 64295 Darmstadt (DE); GRYCZKE, Andreas, 64347 Griesheim (DE)
(74) Vertreter: Gottschalk, Michael
(86) Internationale Anmeldenummer: PCT/EP2003/000935
(87) Internationale Veröffentlichungsnummer: WO 2003/072083

(56) Entgegenhaltungen:
- WO-A-00/35450
- US-A1- 2001 031 278
- US-A1- 2001 036 476

## Beschreibung

### Gebiet der Erfindung

Die Erfindung betrifft ein Verfahren zur Herstellung von geschmacksisolierten, wirkstoffhaltigen Granulaten oder Pulvern durch Aufschmelzen einer Mischung eines (Meth)acrylat-Copolymers mit anionischen Gruppen und eines pharmazeutischen Wirkstoffstoffs, Extrusion der Mischung und Zerkleinern des Extrudats zu einem Granulat oder Pulver.

### Stand der Technik

EP-A 0 417 588 beschreibt ein Verfahren zur Herstellung eines komplexierten Arzneimittels aus einem ionogenen Wirkstoff durch Umsetzung des Wirkstoffs mit einem komplementär ionogenen, partikelförmigen Polymer in Gegenwart einer zum Anfeuchten der Mischung ausreichenden Wassermenge. Bei Wirkstoffssalzen ist es erforderlich, der Mischung eine Säure oder Base zur Neutralisation des Gegenions des Wirkstoffs zuzusetzen. Im Falle der Umsetzung von Wirkstoffsalzen wie Propranolol-HCl, Verapamil-HCI oder Metoclopramid-HCl mit anionischen (Meth)acrylat-Copolymeren wie EUDRAGIT® L oder EUDRAGIT® L100-55 wird der Mischung beispielsweise Natriumcarbonat zugesetzt. Durch die Umsetzung des Wirkstoffs mit dem komplementär ionogenen Polymer kann allgemein eine Geschmacksisolierung bitter schmeckender Wirkstoffe erreicht werden.

WO 01/43935 beschreibt ein Verfahren zur Herstellung von Formkörpern mittels Spritzguß durch Aufschmelzen einer Mischung aus einem (Meth)acrylat-Copolymeren, das sich aus 40 bis 100 Gew.-% radikalisch polymerisierten C₁-bis C₄-Alkylestern der Acryl- oder der Methacrylsäure und 0 bis 60 Gew.-% (Meth)acrylat-Monomeren mit einer anionischen Gruppe im Alkylrest zusammensetzt, das 0,1 bis 3 Gew.-% eines Trennmittels enthält, wobei gegebenenfalls 0 bis 50 Gew.-% eines Trockenstellmittels, 0 bis 30 Gew.-% eines Weichmachers, 0 bis 100 Gew.-% Additive oder Hilfsstoffe, 0 bis 100 Gew.-% eines pharmazeutischen Wirkstoffs, 0 bis 20 Gew.-% eines weiteren Polymeren oder Copolymeren in der Mischung enthalten sein können, wobei die Mischung vor dem Aufschmelzen einen Gehalt an niedrigsiedenden Bestandteilen mit einem Dampfdruck von mindestens 1,9 bar bei 120°C von über 0,5 Gew.-% aufweist.

In einem weiteren Verfahrensschritt erfolgt ein Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird. Anschließend erfolgt ein Einspritzen der aufgeschmolzenen und entgasten Mischung in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, das Abkühlen der Schmelzemischung und Entnahme des erhaltenen Formkörpers aus der Form. Das Verfahren ist allgemein geeignet zur Verarbeitung einer Vielzahl von Wirkstoffen, wie z. B. Verapamil, und deren pharmazeutisch verwendeten Salzen.

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

Die Mindestfilmbildungstemperatur (MFT nach DIN 53 778) liegt im Bereich zwischen 0 und 25 °C, so daß die Verarbeitung bei Raumtemperatur ohne Weichmacherzusatz möglich ist. Die Reißdehnung der Filme, gemessen nach DIN 53 455, liegt bei einem Gehalt von höchstens 10 Gew.-% Triethylcitrat in der Regel bei 50 % oder mehr. Die Mischpolymerisate lassen sich mittels Schmelzextrusion z. B. zu Filmen verarbeiten, in die später Arzneimitteikerne eingebracht werden. Die Möglichkeit der unmittelbaren Einarbeitung von Wirkstoffen während der Extrusion wird nicht erwogen.

WO 00/35450 A offenbart die Herstellung von methylphenidat-haltigen Formulierungen mit kontrollierter Wirkstofffreisetzung durch Schmelzextrusion.

### Aufgabe und Lösung

Bei der Herstellung von Arzneiformen ist man In der Regel bestrebt die Zahl der Einsatzstoffe gering zu halten und auch mengenmäßig Zusätze von Hilfsstoffen wie z. B. Weichmacher oder Trennmittel gering zu halten oder nach Möglichkeit Völlig zu vermeiden. Dadurch können etwaige Unverträglichk.eitsprobleme, unerwünschte Wechselwirkungen oder sonstige Unwägbarkeiten gering gehalten oder von vom herein- ausgeschlossen werden. Für diesen Ansatz bietet das in der EP-A 0 417 588 beschriebene Verfahren einen guten Ansatz, da die Arzneiformen in der Regel nur den Wirkstoff und ein komplementär ionogenes Polymer enthalten.

Gemäß EP-A 0 417 588 kann eine Geschmacksisolieruhg bitter schmeckender Wirkstoffe durch Umsetzung eines Wirkstoffs mit einem komplementär ionogenen, partikelförmigen Polymer in Gegenwart einer zum Anfeuchten der Mischung ausreichenden Wassermenge erreicht werden. Die erhaltenen körnigen Massen werden anschließend durch Trocknung in wirkstoffhaltige Pulver oder Pasten überführt. Der Zusatz von weiteren pharmazeutischen Hilfsstoffen ist dabei in der Regel nicht erforderlich mit der Ausnahme, daß bei Wirkstoffssalzen der Mischung eine Säure oder Base zur Neutralisation des Gegenions des Wirkstoffs zuzusetzen ist.

Ais unerwünschter Nebeneffekt stellt sich bei basischen Wirkstoffsalzen mit saurem Gegenion wie z. B. Verapamil-HCI durch die Neutralisation mit einer Base ein leicht alkalischer pH-Wert ein, der einen unerwünschten seifenähnlichen Geschmack hervorruft. Zudem kann der alkalische pH-Wert dazu führen, daß mit der Zeit unerwünschte Wechselwirkungen mit dem Wirkstoff eintreten und die Langzeit-Lagerstabilität der erhaltenen Arzneiformen beeinträchtigt wird.

Ausgehend von dieser Problematik wurde es als eine Aufgabe gesehen, eine Geschmacksisolierung für basische Wirkstoffsalze bereitzustellen, bei der ein Zusatz von Hilfsstoffen allgemein und insbesondere von basisch wirkenden Substanzen vermieden werden kann. Die Herstellung sollte gegenüber der EP-A 0 417 588 vereinfacht sein. Die resultierenden Formulierungen der geschmacksisolierten Wirkstoffe sollen darüberhinaus eine vergleichsweise verbesserte Langzeit-Lagerstabilität aufweisen.

Das Verfahren gemäß der WO 01/43935 ist dabei nicht geeignet, da bei der Spritzgußverarbeitung zwingend der Zusatz von Trennmitteln erforderlich ist. Bei der Verarbeitung von (Meth)acrylat-Copolymeren mit höheren Gehalten von anionischen Resten, wie Sie für eine Wirkstoffkomplexierung als notwendig angesehen werden, ist zudem der Zusatz von Weichmachern unvermeidbar.

Die Aufgabe wird gelöst durch ein
Verfahren zur Herstellung von wirkstoffhaltigen Granulaten oder Pulvern durch die Schritte
a) Aufschmelzen einer Mischung eines pharmazeutischen Wirkstoffs und eines (Meth)acrylat-Copolymers, das aus 40 bis 75 Gew.-% radikalisch polymerisierten C₁- bis C₄-Alkylestem der Acryl- oder der Methacrylsäure und 25 bis 60 Gew.-% (Meth)acrylat-Monomeren mit einer anionischen Gruppe im Alkylrest besteht,
b) Extrusion der Mischung
c) Zerkleinern des Extrudats zu einem Granulat oder Pulver
dadurch gekennzeichnet, dass
**die Verarbeitungstemperatur 0 bis 100 °C über der Glastemperatur T_{mg} (ohne Weichmacherzusatz, nach ISO 11357-2, Punkt 3.3.3) des (Meth)acrylat-Copolymers liegt und** der Wirkstoff das Salz einer basischen Substanz ist und dass der am erhaltenen Pulver oder Granulat messbare pH-Wert pH 7,0 oder weniger als pH 7,0 beträgt.

Es wurde nun gefunden, dass das Salz einer basischen Substanz auf dem Wege der Schmelzzubereitung durch Extrusion in Mischung mit einem (Meth)acrylat-Copolymer mit anionischen Resten ohne den Zusatz von basisch wirkenden Substanzen zu geschmacksisolierten Granulaten oder Pulvern verarbeitet werden können. Dadurch kann der pH-Wert der erhaltenen Pulvers oder Granulats pH 7,0 oder weniger als pH 7,0 betragen, so dass eine für die Langzeit-Lagerstabilität des Wirkstoff günstigere Umgebung erhalten wird. Hohe Mengen weiterer Zusatzstoffe, z. B. Weichmacher oder Trennmittel, wie Sie für eine Spritzgussverarbeitung notwendig wären, können vermieden werden.

### Ausführung der Erfindung

Die Erfindung betrifft ein Verfahren zur Herstellung von wirkstoffhaltigen Granulaten oder Pulvern durch die Schritte
a) Aufschmelzen einer Mischung eines pharmazeutischen Wirkstoffs und eines (Meth)acrylat-Copolymers, das aus 40 bis 75 Gew.-% radikalisch polymerisierten C₁- bis C₄-Alkylestern der Acryl- oder der Methacrylsäure und 25 bis 60 Gew.-% (Meth)acrylat-Monomeren mit einer anionischen Gruppe im Alkylrest besteht,
b) Extrusion der Mischung
c) Zerkleinern des Extrudats zu einem Granulat oder Pulver
dadurch gekennzeichnet, dass
die Verarbeitungstemperatur 0 bis 100 °C über der Glastemperatur *T*_{mg} (ohne Weichmacherzusatz, nach ISO 11357-2, Punkt 3.3.3) des (Meth)acrylat-Copolymers liegt und der Wirkstoff das Salz einer basischen Substanz ist und dass der am erhaltenen Pulver oder Granulat messbare pH-Wert pH 7,0 oder weniger als pH 7,0 beträgt.

### (Meth)acrylat-Copolymere mit anionischen Resten

Das (Meth)acrylat-Copolymere besteht zu 40 bis 80, bevorzugt zu 45 bis 75, insbesondere zu 55 bis 65 Gew.-% aus radikalisch polymerisierten C₁- bis C₄-Alkylestem der Acryl- oder der Methacrylsäure und zu 20 bis 60, bevorzugt 25 bis 58, insbesondere 45 bis 55 Gew.-% (Meth)acrylat-Monomeren mit einer anionischen Gruppe im Alkylrest enthalten.

In der Regel addieren sich die Anteile zu 100 Gew.-%. Es versteht sich jedoch, daß zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere enthalten sein können.

C₁- bis C₄-Alkylester der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein.

### Typen EUDRAGIT® L oder EUDRAGIT® L100-55

Geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L (50 Gew.-% Methylmethacrylat und 50 Gew.-% Methacrylsäure) oder EUDRAGIT® 1.100-55 (50 Gew.-% Ethylacrylat und 50 Gew.-% Methacrylsäure)).

### Typ EUDRAGIT® S

Geeignet sind anionische (Meth)acrylat Copolymere aus 20 bis 40 Gew.-% Methacrylsäure und 80 bis 60 Gew.-% Methylmethacrylat (Typ EUDRAGIT® S).

### Typ EUDRAGIT® mit mittlerem Gehalt an Methacrylsäure.

Geeignet sind ebenfalls anionische (Meth)acrylat Copolymere aus 20 bis 34 Gew.-% Methacrylsäure und/oder Acrylsäure, 20 bis 69 Gew.-% Methylacrylat und 0 bis 40 Gew.-% Ethylacrylat und gegebenenfalls 0 bis 10 Gew.-% weiteren vinylisch copolymerisierbaren Monomeren, mit der Maßgabe, daß die Glastemperatur des Copolymers nach ISO 11357-2, Punkt 3.3.3, höchstens 60 °C beträgt.

Das Copolymer setzt sich insbesondere zusammen aus radikalisch polymerisierten Einheiten von
20 bis 34, bevorzugt 25 bis 33, besonders bevorzugt 28 bis 32 Gew.-% Methacrylsäure oder Acrylsäure, bevorzugt ist Methacrylsäure,
20 bis 69, bevorzugt 35 bis 65, besonders bevorzugt 35 bis 55 Gew.-% Methylacrylat und gegebenenfalls
0 bis 40, bevorzugt 5 bis 35, besonders bevorzugt 15 bis 35 Gew.-% Ethylacrylat zusammen, mit der Maßgabe, daß die Glastemperatur des Copolymers (ohne Weichmacherzusatz) nach ISO 11357-2, Punkt 3.3.3, höchstens 60, bevorzugt 40 bis 60, besonders bevorzugt 45 bis 55 °C beträgt.

Das Copolymer besteht bevorzugt in wesentlichen bis ausschließlich aus den Monomeren Methacrylsäure, Methylacrylat und Ethylacrylat in den oben angegebenen Mengenanteilen. In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

### Herstellung der (Meth)acrylat-Copolymere

Die (Meth)acrylat-Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den gewünschten Teilchengrößenbereich gebracht werden. Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Basische Wirkstoffsalze

Das basische Wirkstoffsalze kann z. B. Verapamil-HCI sein. Bevorzugt liegt der pH des resultierenden Verapamil-haltigen Granulats oder Pulvers bei pH 2,3 bis pH 4,5.

### Verarbeitung durch Schmelzextrusion

Die Herstellung von wirkstoffhaltigen Granulaten oder Pulvern erfolgt durch die Schritte
a) Aufschmelzen einer Mischung eines pharmazeutischen Wirkstoffs und eines (Meth)acrylat-Copolymers, das aus 40 bis 75 Gew.-% radikalisch polymerisierten C₁- bis C₄-Alkylestern der Acryl- oder der Methacrylsäure und kann 25 bis 60 Gew.-% (Meth)acrylat-Monomeren mit einer anionischen Gruppe im Alkylrest besteht
b) Extrusion der Mischung, bevorzugt in einem Extruder, z. B. einem Zweischneckenextruder mit gleichläufigen oder gegenläufigen Schnecken
c) Zerkleinern des Extrudats zu einem Granulat oder Pulver, z. B durch Heißabschlag des Extrudats bzw. Zermahlen des Granulats.

### Mengenverhältnisse

Das Mengenverhältnis von Wirkstoff zu (Meth)acrylat-Copolymer kann z. B. 10 zu 1 bis 1 zu 10 , bevorzugt 10 zu 2 bis 2 zu 10, besonders bevorzugt 10 zu 3 bis 3 zu 10 bezogen auf Gewichtsteile betragen.

### Hilfsstoffe

Das erfindungsgemäße Verfahren ermöglicht es, die Zahl und Mengenanteile ansonsten üblicher pharmazeutischer Hilfsstoffe zu verringern oder ganz auf diese zu verzichten. Sofern überhaupt Zusatzstoffe verwendet werden sollen, können diese der Mischung (Verfahrensschritt a)) vor oder nach dem Aufschmelzen zugesetzt werden oder auch während der Extrusion (Verfahrensschritt b)) im Extruder zugegeben werden.

### Trennmittel (Formtrennmittel)

Bevorzugt wird der Mischung kein oder weniger als 0,1 Gew.-% von Trennmitteln zugesetzt werden.

### Beispiele für Trennmittel (Formtrennmittel) sind:

Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind z. B. Glycerolmonostearat, Stearylalkohol, Glycerolbehensäureester, Cetylalkohol, Palmitinsäure, Kanaubawachs, Bienenwachs.

### Weichmacher:

Bevorzugt wird der Mischung kein oder weniger als 0,5 Gew.-% Weichmacher zugesetzt werden.

Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele für Weichmacher sind Citronensäurealkylester, Propylenglykol, Glycerinester, Phtalsäurealkylester, Sebacinsäurealkylester, Sucroseester, Sorbitanester, Diethylsebacat , . Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat.

### Trockenstellmittel (Antihaftmittel)

Bevorzugt wird der Mischung kein oder weniger als 0,1 Gew.-% von Trockenstellmittel (Antihaftmittel) zugesetzt werden.

Trockenstellmittel haben folgende Eigenschaften: sie verfügen über große spezifische Oberflächen, sind chemisch inert, sind gut rieselfähig und feinteilig. Aufgrund dieser Eigenschaften lassen sie sich vorteilhaft in Schmelzen homogen verteilen und erniedrigen die Klebrigkeit von Polymeren, die z. B. aufgrund höherer Gehalte an anionischen Resten zur Klebrigkeit neigen.

Beispiele für Trockenstellmittel sind: Aluminiumoxid, Magnesiumoxid, Kaolin, Talkum, Kieselsäure (Aerosile), Bariumsulfat, Ruß und Cellulose.

### Weitere pharmazeutisch übliche Hilfsstoffe

Bevorzugt werden der Mischung keine oder weniger als 10 Gew.%, bevorzugt weniger als 2 Gew.-% von den nachstehend genannten weiteren pharmazeutisch üblichen Hilfsstoffen, wie Stabilisatoren, Farbstoffe, Antioxidantien, Netzmittel, Pigmente und Glanzmittel zugesetzt.

### Verarbeitungstemperaturen

Die Verarbeitungstemperatur bei den Verfahrensschritten a) und b) kann im Bereich von 50 bis 200 °C, bevorzugt im Bereich von 80 bis 180 °C liegen. Je nach eingesetztem Copolymer liegt die Verarbeitungstemperatur 0 bis 100, besonders bevorzugt 10 bis 50 °C über dessen Glastemperatur *T*_{mg} (ohne Weichmacherzusatz, nach ISO 11357-2, Punkt 3.3.3).

### Granulate und Pulver

Nach dem erfindungsgemäßen Verfahren sind wirkstoffhaltige Granulate oder Pulver herstellbar, die den basischen Wirkstoff in komplexierter Form enthalten. Der genaue molekulare Mechanismus der Komplexierung bzw. die exakte Struktur der Komplexe sind nicht bekannt.

In dem erfindungsgemäß hergestellten Trockenprodukt liegt der enthaltene Wirkstoff weitgehend, in der Regel zu mehr als 90 %, bevorzugt zu mehr als 95 %, insbesondere zu mehr als 97 % in Form eines Komplexes mit dem Polymer vor. Der Anteil des nicht komplex gebundenen Wirkstoffes lässt sich bestimmen, wenn man das Produkt in Wasser aufnimmt, filtriert und den Wirkstoffgehalt im Filtrat ermittelt. Der nicht komplexierte Anteil kann erforderlichenfalls auf die gleiche Weise aus dem Produkt entfernt werden.

Die wirkstoffhaltigen Granulate oder Pulver enthalten kein oder weniger als 0.1 Gew.-% an Trennmitteln.

Die wirkstoffhaltigen Granulate oder Pulver enthalten kein oder weniger als 0,5 Gew.-% an Weichmachern.
Die wirkstoffhaltigen Granulate oder Pulver enthalten kein oder weniger als 0,1 Gew.-% an Trockenstellmittein (Antihaftmittel).
Die wirkstoffhaltigen Granulate oder Pulver enthalten keine oder weniger als 10 Gew.-% weitere pharmazeutisch übliche Hilfsstoffe, wie Stabilisatoren, Farbstoffe, Antioxidantien, Netzmittel, Pigmente und Glanzmittel.

Granulate können z. B. durch Heißabschlag von Extrudatsträngen erhalten werden. Die mittleren Teilchengrößen der Granulate liegen bevorzugt im Bereich von 1 bis 5 mm.

Pulver können durch Mahlen von Granulat, z. B. in Stiftmühlen, erhalten werden. Die mittleren Teilchengrößen der Pulver liegen bevorzugt im Bereich von 1 bis 1000 µm.

### PH-Werte

Der an den erhaltenen Pulvern oder Granulaten messbare pH-Wert liegt bei pH 7,0 oder bei weniger als pH 7,0, bevorzugt beträgt der pH-Wert 2,5 bis 6,0, besonders bevorzugt 3 bis 5.
Wenn als Wirkstoff-Salz Verapamil-HCl eingesetzt wird, soll der pH-Wert des Granulats oder Pulvers bevorzugt pH 2,3 bis pH 4,5, besonders bevorzugt pH 2,5 bis 3,5 betragen.

Die pH-Werte können z. B. in einer 1% igen Suspension in gereinigtem Wasser bei Raumtemperatur mit einem pH- Messstäbchen oder einer pH-Messelektrode gemessen werden.

### Bitterwerte

Bitterwerte können nach DAB 1999 Methode 2.8.N8 (Bestimmung des Bitterwertes) bestimmt werden. Die an den Pulver und Granulaten gemessenen Bitterwerte liegen im Bereich zwischen 1000 und 2000, bevorzugt bei weniger als 1000.

Die Maskierung des unangenehmen, insbesondere bitteren Geschmackes eines Wirkstoffes ist eines der wesentlichen Ziele des Verfahrens. Als Maß für die Geschmacksintensität kann der Bitterwert nach DAB 1999 Methode 2.8.N8 (Bestimmung des Bitterwertes) herangezogen werden. Die Verminderung der bitteren Geschmackempfindung läuft mit dem gebundenen Anteil ungefähr proportional; während z.B. reines Verapamil-HCl einen Bitterwert über 100.000 hat, liegt der Wert für den Komplex mit 90 % gebundenem Wirkstoff unter 5000 und mit 97 % gebundenem Wirkstoff unter 1000. Ein Bitterwert von 1000 ist für pharmazeutische Praxis in der Regel ausreichend. Der dafür erforderliche Grad der Komplexierung hängt vom Bitterwert des reinen Wirkstoffes und von seiner Konzentration in der Darreichungsform ab.

### Wirkstoffe

Die erfindungsgemäß zu verwendenden Wirkstoffe sind Salze basischer Substanzen. Die Erfindung kann vorteilhaft für die folgenden basischen Substanzen in Salzform, bevorzugt für solche mit bitterem Geschmack, d. h. einem Bitterwert von mindestens 100, bevorzugt 1000, insbesondere 5000 gemessen am reinen Wirkstoff, aus den angegebenen Wirkstoffklassen angewendet werden.

Die erfindungsgemäß einsetzbaren Wirkstoffe, die Salze einer basischen Substanzen ist, können z. B. zur Wirkstoffklasse der Analgetika, Antirheumatika, Psychopharmaka, Antibiotika, Betablocker, Antidiabetika, H1 Antihistaminika, H2 Antihistaminika und/oder Vitamine gehören.

Innerhalb dieser Wirkstoffklassen sind insbesondere die folgenden Wirkstoffe zu nennen:

### Analgetika und Antirheumatika:

Levacetylmethadol hydrochlorid, Oxycodon hydrochlorid, -hydrochlorid-3-Wasser, Tramadol hydrochlorid, Tilidin hydrochlorid

### Psychopharmaka:

Prometazin embonat; -hydrochlorid, -teoclat, Donepezil hydrochlorid, Nefazodon hydrochlorid, Reboxetin mesilat, Sertralin hydrochlorid

### Antibiotika:

Erythromycin-acistrat, Erythromycin-estolat, Erythromycin-ethylsuccinat, Erythromycin-glucoheptonat, Erythromycin-lactobionat, Erythromycin-propionat, Erythromycin-stearat, Erythromycin-stinoprat, Grepafloxacin hydrochlorid, Ciprofloxacin hydrochlorid-1-Wasser, Levofloxacin hydrochlorid, -lactat, Trovafloxacin mesylat, Nevirapin hydrochlorid, Chlorhexidin acetat, -diacetat-hydrat; -dihydrochlorid, -digluconat, -gluconat, Metronidazol benzoat, Tetracyclin hydrochlorid, -phosphat, Chlortetracyclin hydrochlorid, Oxytetracyclin hydrochlorid, Doxycyclin hyclat, Minocyclin hydrochlorid-hydrat, Neomycin sulfat, Tobramycin sulfat, Clindamycin hydrochlorid, Moxifloxacin hydrochlorid, Indinavir sulfat, Saquinavir mesylat, Nelfinavir mesylat, Amatidin hydrochlorid, Streptomycin sulfat,Amikacin-bis-hydrogensulfat, Paromomycinsulfat, Tobramycinsulfat

### Betablocker

Propanolol hydrochlorid, Metoprolol tartrat, -fumarat, Bisoprolol fumarat , Nebivolol hydrochlorid, Betaxolol hydrochlorid, Tertatolol hydrochlorid, Bopindolol malonat, Esmolol hydrochlorid, Oxprenolol hydrochlorid

### Antidiabetika:

Metformin hydrochlorid, Pioglitazon hydrochlorid, Rosiglitazon maleat

### H1 Antihistaminika:

Diphenhydramin acefyllinat, -citrat, -hydrochlorid, -mesilat, Fexofenadin hydrochlorid

### H2 Antihistaminika:

Cimetidin hydrochlorid, Ticlopidin hydrochlorid, Ranitidin hydrochlorid, Roxatidin acetat

### Vitamine:

Thiamin disulfid, -disulfid-O,O-dinicotinat, -hydrobromid, -hydrochlorid, -nitrat

### Weitere:

Chinidin gluconat; hydrogensulfat-4-Wasser; -lactat, -nitrit, -polygalacturonat, - sulfat, -sulfat-2-Wasser, Amiloprilose, Pseudoephedrin hydrochlorid, Sildenafil citrat, Granisetron hydrochlorid, Chinin sulfat-2-Wasser; -monohydrochlorid -2-Wasser, Metoclopramid dihydrochlorid-1-Wasser; -hydrochlorid, Pentoxyverin dihydrogencitrat; -hydrochlorid

Beispiele für bitter schmeckende Wirkstoffe, die im basischen bis leicht basischen Milieu (oberhalb pH 7) Instabilitäten zeigen:
Erythromycin-estolat, Chlortetracyclin-hydrochlorid, Ranitidin-hydrochlorid, Streptomycin sulfat, Amikacin-bis(hydrogensulfat), Neomycin sulfat Paromomycinsulfat, Tobramycinsulfat

### Verwendung

Die nach dem erfindungsgemäßen Verfahren herstellbaren wirkstoffhaltige Granulate oder Pulver können zur Herstellung von Arzneiformen verwendet werden, bevorzugt zur Geschmacksisolierung.

Beispiele für Arzneiformen sind: Pellets, Tabletten oder Sachets mit und ohne kontrollierter Wirkstoffabgabe, flüssige Arzneiformen, z. B. Sirupe, Tropfen, Suspensionen. Diese Arzneiformen können weitere übliche pharmazeutische Hilfsstoffe, die jedoch so ausgewählt werden müssen, dass unerwünschte ionische Interaktionen mit dem erfindungsgemäßen Pulver oder Granulat ausgeschlossen sind, weil sie den Bitterwert schon in der Arzneiform, z. B. während der Lagerung erhöhen können.

### Beispiele

Für die Herstellung des Hotmelt-Compounds wird ein 18 mm, gleichläufiger, Doppelschneckenextruder mit einer Baulänge von 40 D verwendet (Typ Micro 18 GL-40D Pharma, Fa. Leistritz Extrusionstechnik GmbH, Nürnberg). Die Zuführung der pulverförmigen Rohstoffe erfolgt einzeln über kontinuierlich, gravimetrisch arbeitende Dosiereinrichtungen. Der Düsendurchmesser beträgt 1,5 mm. Der Extruder ist unterteilt in 9 separat temperierbare Zylinder. Der Einzugszylinder wird grundsätzlich gekühlt, so dass die Temperatur unter 10 °C bleibt. Im 4. Zylinder befindet sing eine Zuführöffnung, die für die ggf. notwendige Zugabe von Flüssigkeiten genutzt wird. Grundsätzlich ist die Anordnung der Zylinder frei wählbar. Die erhaltenen Stränge werden auf einem Abzugsband mit Luft gekühlt und mittels eines Stranggranulators (Typ RCP-2.0, Fa. Randcastle Extrusions Systems Inc., Cedar Grove, NY, USA) zu zylindrischen Granulatkörnem mit einem Durchmesser von ca. 1,5 mm und einer Länge von 2-3 mm geschnitten.

Das Vermahlen des Granulates erfolgt in einer Analysenmühle (Typ A10, IKA-Labortechnik, Janke & Kunkel GmbH & Co. KG, Staufen, Deutschland) über eine Zeit von ca. 1 min. Anschließend wird der Feinanteil unter 315 µm abgetrennt und für weitere Tests eingesetzt.

Der Bitterwert wird nach DAB 1999, 2.8:N8 (Bestimmung des Bitterwertes) ohne Einbezug des persönlichen Korrekturfaktors ermittelt.
Zugfestigkeit der Tabletten: Bruchfestigkeitstester (Fa. Pharmatest)
Zerfall der Tabletten: DAB 2000
Wirkstoff Freigabe nach USP 24 NF 19 mit 50 Rpm in HCl.

### Beispiel 1: Geschmacksisolierung eines kationischen Wirkstoffsalzes bei einem molaren Verhältnis der funktionellen Gruppen im Polymer zum Verapamil von 1 mol zu 0,38 mol

Ein Hotmelt-Compound wird hergestellt aus 50 Masseteilen Verapamil HCI und 50 Masseteilen **EUDRAGIT^{®} L 100-55** (Copolymer aus 50 Gew.-% Ethylacrylat und 50 Gew.-% Methacrylsäure). Die Temperatur der Extruderzylinder wurde zwischen 120°C und 130 °C gehalten. Die Drehzahl der Schnecken lag bei 300/min, der Durchsatz betrug ca. 700 g/Std. Die mittlere Verweilzeit des Materials im Extruder betrug ca. 4 min. Das aus diesem Extrusionsprozeß hergestellte Granulat ist leicht gelblich gefärbt und klar bis leicht milchig getrübt. 10 Granulatkörner hinterlassen im Mund nach 30 Sekunden nur einen leicht bitteren Geschmack. Für das gemahlene Pulver wird ein Bitterwert von unter 10 bestimmt. Dies entspricht einer Reduktion des Bitterwertes zum reinen Verapamil-HCl um eine 10er Potenz.
Werden vom gemahlenen Pulver 800 mg in 50 mL vollentsalztem Wasser eingerührt (entsprechend 8 mg Verapamil pro Milliliter Wasser), so wird ein pH-Wert von 3 ermittelt.

### Beispiel 2: Herstellung von geschmacksneutralen Verapamil Tabletten:

160 g des in Beispiel 1 beschriebenen, gemahlenen Hotmelt-Compounds, 230 g Lactose D20, 180 g Avicel PH 102, 30 g Explotab^{®} wurden 15 min in einem Doppelkonusmischer mit einer Drehzahl von ca. 30/min gemischt und durch ein Sieb mit 1 mm Maschenweite gegeben. Danach werden 3 g Magnesiumstearat zugegeben und weitere 5 min gemischt.
Die Pulvermischung wird auf einer 1-Stempel-Exzenterpresse (Typ EK0, Fa. Korsch, Berlin) mit einem Druck von ca. 155 MPa zu Tabletten verpresst (35 Tabletten / min). Das Sollgewicht wird auf 603 mg (entsprechend 80 mg Verapamil HCl) eingestellt.
Eigenschaften der Tabletten:
Durchmesser 11,1 mm
Tensile Strength: ca. 2,37 N/mm²
Gewicht 600 mg (s: 3mg)

Die erhaltenen Tabletten hinterlassen nach 10 s Speichelbenetzung im Mund keinen bitteren Geschmack. Die Wirkstoffabgabe betrug nach 30 min ca. 28 % und nach 60 min. ca. 54 %

### Beispiel 3: Geschmacksisolierung eines kationischen Wirkstoffsalzes bei einem molaren Verhältnis der funktionellen Gruppen im Polymer zum Verapamil von 1 mol zu 0,3 mol

Ein Hotmelt-Compound wird hergestellt aus 30 Masseteilen Verapamil-HCI und 70 Masseteilen eines Copolymers aus 40 Gew.-% Methylacrylat, 30 Gew.-% Ethylacrylat und 30 Gew.-% Methacrylsäure. Die Temperatur der Extruderzylinder wurde zwischen 140°C und 150 °C gehalten. Die Drehzahl der Schnecken lag bei 136/min, der Durchsatz betrug ca. 600 g/Std. Die mittlere Verweilzeit des Materials im Extruder betrug ca. 4 ½ min. Das aus diesem Extrusionsprozeß hergestellte Granulat ist farblos bis leicht gelblich gefärbt und klar. 10 Granulatkörner hinterlassen im Mund nach 30 s einen neutralen Geschmack. Für das gemahlene Pulver wird ein Bitterwert von unter 0,1 bestimmt. Dies entspricht einer Reduktion des Bitterwertes zum reinen Verapamil HCl um drei 10er Potenzen.

### Beispiel 4: Herstellung von geschmacksneutralen Verapamil -HCl Tabletten:

266,66 g des in Beispiel 5 beschriebenen, gemahlenen Hotmelt-Compounds, 123,34 g Lactose D20,180 g Avicel PH 102, 30 g Explotäb^{®} wurden 15 min in einem Doppelkonusmischer mit einer Drehzahl von ca. 30/min gemischt und durch ein Sieb mit 1 mm Maschenweite gegeben. Danach werden 3 g Magnesiumstearat zugegeben und weitere 5 min gemischt.
Die Pulvermischung wird auf einer 1-Stempel-Exzenterpresse (Typ EK0, Fa. Korsch, Berlin) mit einem Druck von ca. 155 MPa zu Tabletten verpresst (35 Tabletten / min). Das Sollgewicht wird auf 603 mg (entsprechend 80 mg Verapamil KCl)eingestellt.
Eigenschaften .der Tabletten:
Durchmesser 11,1mm
Tensile Strength: ca. 1,14 N/mm²
Gewicht 595 mg (s: 5mg)

Die erhaltenen Tabletten hinterlassen nach 10 s Speichelbenetzung im Mund einen neutralen Geschmack. Die Wirkstoffabgabe betrug nach 30 min ca. 36 % und nach 60 min. ca. 55 %

### Beispiel 5: Geschmacksisolierung eines kationischen Wirkstoffsalzes bei einem molaren Verhältnis der funktIonellen Gruppen im Polymer zum Verapamil von 1 mol zu 0,42 mol

Ein Hotmeit-Compound wird hergestellt aus 40 Masseteilen Verapamil HCl und 60 Masseteilen eines Copolymers aus 40 Gew.-% Methylacrylat, 30 Gew.-% Ethylacrylat und 30 Gew.-% Methacrylsäure. Die Temperatur der Extruderzylinder wurde zwischen 140°C und 150 °C gehalten. Die Drehzahl der Schnecken lag bei 150/min, der Durchsatz betrug ca. 600 g/Std. Die mittlere Verweilzeit des Materials im Extruder betrug ca. 4½ min. Das aus diesem Extrusionsprozeß hergestellte Granulat ist farblos bis leicht gelblich gefärbt und klar. 10 Granulatkörner hinterlassen im Mund nach 30 s einen neutralen Geschmack. Für das gemahlene Pulver wird ein Bitterwert von unter 10 bestimmt. Dies entspricht einer Reduktion des Bitterwertes zum reinen Verapamil HCl um eine 10er Potenz.
Werden vom gemahlenen Pulver 800 mg in 50 mL vollentsalztem Wasser eingerührt (entsprechend 8 mg Verapamil-HCl pro Milliliter Wasser), so wird ein pH-Wert von -3,8 ermittelt.

### Beispiel 6: Herstellung von geschmacksneutralen Verapamil Tabletten:

200 g des in Beispiel 7 beschriebenen, gemahlenen Hotmelt-Compounds, 190 g Lactose D20, 180 g Avicel PH 102, 30 g Explotab^{®} wurden 15 min in einem Doppelkonusmischer mit einer Drehzahl von ca. 30/min gemischt und durch ein Sieb mit 1mm Maschenweite gegeben. Danach werden 3g Magnesiumstearat zugegeben und weitere 5 min gemischt.
Die Pulvermischung wird auf einer 1-Stempel-exzenterpresse (Typ EK0, Fa. Korsch, Berlin) mit einem Druck von ca. 155 MPa zu Tabletten verpresst (35 Tabletten / min). Das Soligewicht wird auf 603 mg (entsprechend 80 mg Verapamil-HCl) eingestellt.
Eigenschaften der Tabletten:
Durchmesser 11,1mm
Tensile Strength: ca.1,26 N/mm²
Gewicht 586 mg (s: 4mg)

Die erhaltenen Tabletten hinterlassen nach 10 s Speichelbenetzung Im Mund einen neutralen Geschmack. Die Wirkstoffabgabe betrug nach 30 min ca. 38 % und nach 60 min. ca. 59 %

### Beispiel 7: Geschmacksisolienrng eines kationischen Wirkstoffsalzes bei einem molaren Verhältnis der funktionellen Gruppen im Polymer zum Verapamil von 1 mol zu 0,63 mol

Ein Hatrnelt-Compound wird hergestellt aus 50 Masseteilen Verapamü-HCI und 50 Masseteilen eines Copolymers aus 40 Gew.-% Methylacrylat, 30 Gew.-% Ethylacrylat und 30 Gew.-% Methacrylsäure. Die Temperatur der Extruderzylinder wurde zwischen 140°C und 150 °C gehalten. Die Drehzahl der Schnecken lag bei 150/min, der Durchsatz betrug ca. 600 g/Std. Die mittlere Verweilzeit des Materials im Extruder betrug ca. 4½ min. Das aus diesem Extrusionsprozeß hergestellte Granulat ist farblos bis leicht gelblich gefärbt und klar. 10 Granulätkörner hinterlassen im Mund nach 30 s einen neutralen Geschmack. Für das gemahlene Pulver wird ein Bitterwert von unter 10 bestimmt. Dies entspricht einer Reduktion des Bitterwertes zum reinen Verapamil HCl um eine 10er Potenz.
Werden vom gemahlenen Pulver 800 mg in 50 mL vollentsalztern Wasser eingerührt (entsprechend 8 mg Verapamil pro Milliliter Wasser), so wird ein pH-Wert von 4 ermittelt.

### Beispiel 8: Herstellung von geschmacksneirtraien Verapamil Tabletten:

160 g des in Beispiel 9 beschriebenen, gemahlenen Hotmeit-C.ompounds, 230 g Lactose D20, 180 g Avicel, PH 102,30 g Explotab^{®} wurden 15 min in einem Doppolkonusmischer mit einer Drehzahl von ca. 30/min gemischt und durch ein Sieb mit 1 mm Maschenweite gegeben. Danach werden 3 g Magnesiumstearat zugegeben und weitere 5 min gemischt.
Die Pulvermischung wird auf einer 1-Stempel-Exzenterpresse (Typ EK0, Fa. Korsch, Berlin) mit einem Druck von ca. 190 MPa zu Tabletten verpresst (35 Tabletten / min). Das Sollgewicht wird auf 603 mg (entsprechend 80 mg Verapamil HCl)eingestellt.
Eigenschaften der Tabletten:
Durchmesser 11,1 mm
Pressdruck: ca. 190 MPa
Tensile Strength: ca. 2,83 N/mm²
Gewicht 590 mg (s: 4mg)

Die erhaltenen Tabletten hinterlassen nach 10 s Speichelbenetzung im Mund einen neutralen Geschmack. *Die* Wirkstoffabgabe betrug nach 30 min ca. 38 % und nach 60 min. ca. 59 %

### Beispiel 9: Geschmacksisolierung eines kationischen Wirkstoffsalzes bei einem molaren Verhältnis der funktionellen Gruppen im Polymer zum Verapamil von 1 mol zu 0,95 mol

Ein Hotmelt-Compound wird hergestellt aus 60 Masseteilen Verapamil HCI und 40 Massetellen eines Copolymers aus 40 Gew.-% Methylacrylat, 30 Gew.-% Ethylacrylat und 30 Gew.-% Methacrylsäure. Die Temperatur der Extruderzylinder wurde zwischen 140°C und 150 °C gehalten. Die Drehzahl der Schnecken lag bei 150/min, der Durchsatz betrug ca. 600 g/Std. Die mittlere Verweilzeit des Materials im Extruder betrug ca. 4 ½ min. Das aus diesem Extrusionsprozeß hergestellte Granulat ist farblos bis leicht gelblich gefärbt und klar. 10 Granulatkörner hinterlassen im Mund nach 30 s einen leicht bitteren Geschmack. Für das gemahlene Pulver wird ein Bitterwert von unter 10 bestimmt. Dies entspricht einer Reduktion des Bitterwertes zum reinen Verapamil HCl um eine 10er Potenz.
Werden vom gemahlenen Pulver 800 mg in 50 mL vollentsalztem Wasser eingerührt (entsprechend 8 mg Verapamil pro Milliliter Wasser), so wird ein pH-Wert von 4 ermittelt.

### Beispiel 10: Herstellung von geschmacksneutralen Verapamil Tabletten:

133,32 g des in Beispiel 11 beschriebenen, gemahlenen Hotmelt-Compounds, 256,68 g Lactose D20, 180 g Avicel PH 102, 30 g Explotab^{®} wurden 15 min in einem Doppelkonusmischer mit einer Drehzahl von ca. 30/min gemischt und durch ein Sieb mit 1mm Maschenweite gegeben. Danach werden 3 g Magnesiumstearat zugegeben und weitere 5 min gemischt.
Die Pulvermischung wird auf einer 1-Stempel-Exzenterpresse (Typ EK0, Fa. Korsch, Berlin) mit einem Druck von ca. 155 MPa zu Tabletten verpresst (35 Tabletten / min). Das Sollgewicht wird auf 603 mg (entsprechend 80 mg Verapamil HCl)eingestellt.
Eigenschaften der Tabletten:
Durchmesser 11,1mm
Tensile Strength: ca. 1,65 N/mm²
Gewicht 595 mg (s: 3mg)

Die erhaltenen Tabletten hinterlassen nach 10 s Speichelbenetzung im Mund einen leicht bitteren Geschmack. Die Wirkstoffabgabe betrug nach 30 min ca. 56. % und nach 60 min. ca. 77 %.

### Beispiel 11: Geschmacksisolierung eines kationischen Wirktoffsalzes bei einem molaren Verhältnis der funktionellen Gruppen im Polymer zum Verapamil von 1. mol zu 1,47 mol

Ein Hotmeit-Compound wird hergestellt aus 70 Masseteilen Verapamil HCl und 30 Masseteilen eines Copolymers aus 40 Gew.-% Methylacrylat, 30 Gew.-% Ethylacrylat und 30 Gew.-% Methacrylsäure. Die Temperatur der Extruderzylinder wurde zwischen 140°C und 150 °C gehalten. Die Drehzahl der Schnecken lag bei 140/min, der Durchsatz betrug ca. 600 g/Std. Die mittlere Verweilzeit des Materials im Extruder betrug ca. 4 ½ min. Das aus diesem Extrusionsprozeß hergestellte Granulat ist farblos bis leicht gelblich gefärbt und klar. 10 Granulatkörner hinterlassen im Mund nach 30 s einen leicht bitteren Geschmacks Für das gemahlene Pulver wird ein Bitterwert von unter 10 bestimmt. Dies entspricht einer Reduktion des Bitterwertes zum reinen Verapamil HCl um eine 10er Potenz.
Werden vom gemahlenen Pulver 800 mg in 50 mL vollentsalztem Wasser eingerührt (entsprechend 8 mg Verapamil pro Milliliter Wasser), so wird ein pH-Wert von 4 ermittelt.

### Beispiel 12: Herstellung von geschmacksneutralen Verapamil Tabletten:

114,28 g des in Beispiel 13 beschriebenen, gemahlenen Hotmeit-Compounds, 275,72 g Lactose D20,180 g Avicel PH 102, 30 g Explotab^{®} wurden 15 min in einem Doppelkonusmischer mit einer Drehzahl von ca. 30/min gemischt und durch ein Sieb mit 1mm Maschenweite gegeben. Danach werden 3 g Magnesiumstearat zugegeben und wettere 5 min gemischt.
Die Pulvermischung wird auf einer 1-Stempel-Exzenterpresse (Typ EK0, Fa. Korsch, Berlin) mit einem Druck von ca. 155 MPa zu Tabletten, verpresst (35 Tabletten / min). Das Sollgewicht wird auf 603 mg (entsprechend 80 mg Verapamil HCl) eingestellt.
Eigenschaften der Tabletten:
Durchmesser 11,1 mm
Tensile Strength: 2,54 N/mm²
Gewicht 602 mg (s: 3mg)

Die erhaltenen Tabletten hinterlassen nach 10 s Speichelbenetzung im Mund einen deutlich bitteren Geschmack. Die Wirkstoffabgabe betrug nach 30 min ca. 52 % und nach 60 min. ca. 84 %

### Beispiel 13: Herstellung eines geschmacksneutralen Verapamil HCI-Sirups

In einem Rührkessel werden 1279,0 g Saccharose mit 1139,9 g Wasser auf ca. 50 °C erhitzt und bis zur vollständigen Lösung langsam gerührt. Nach dem Abkühlen auf ca. 20 °C gibt man 37,6 g des in Beispiel 9 hergestellten, gemahlenen Compounds zu und homogenisiert ca. 10 Minuten. Zu der Suspension gibt man nun unter Rühren 27,3 g einer Mischung aus microkristalliner Cellulose und Na-Carboxlmethylcellulose (Avicel RC 591^{®}) und 2,8 g Keltrol^{®} F (Polysaccharid B 1459) und homogenisiert ca. 15 Minuten.
Der so hergestellte Sirup ist eine homogene, weiße Flüssigkeit mit süßem Geschmack. Der Soll-Wirkstoffgehalt beträgt 8 mg/L.

## Patentansprüche

1. Verfahren zur Herstellung von varkstoffhaltigen Granulaten oder Pulvern durch die Schritte
a) Aufschmelzen einer Mischung eines pharmazeutischen Wirkstoffs und eines (Meth)acrylat-Capolymers, das aus 40 bis 75 Gew.-% radikalisch polymerisierten C₁- bis C₄-Alkylestern der Acyl- oder der Methacrylsäure und 25 bis 60 Gew.-% (Meth)acrylatMonomeren mit einer anionischen Gruppe im Alkylrest besteht,
b) Extrusion der Mischung,
c) Zerkleinern des Extrudats zu einem Granulat oder Pulver
**dadurch gekennzeichnet, dass**
die Verarbeitungstemperatur 0 bis 100 °C über der Glastemperatur *T*_{mg}, zubestimmen ohne Weichmacherzusatz, nach ISO 11357-2, Punkt 3.3.3, des (Meth)acrylat-Copolymers liegt und der Werkstoff das Salz einer basischen Substanz ist und dass der am erhaltenen Pulver oder Granulat messbare pH-Wert pH 7,0 oder weniger als pH 7,0 betragt.

2. Verfahren nach Anspruch 1, **dadurch gekennzeichnet, daß** der Mischung kein oder weniger als 0,1 Gew.-% von Trennmitteln zugesetzt werden.

3. Verfahren nach Anspruch 1 oder 2, **dadurch gekennzeichnet, daß** der Mischung kein oder weniger als 0,5 Gew.-% Weichmacher zugesetzt werden.

4. Verfahren nach einem oder mehreren der Ansprüche 1 bis 3, **dadurch gekennzeichnet, daß** das Mengenverhältnis von Wirkstoff zu (Meth)acrylat-Copolymer 10 zu 1 bis 1 zu 10 bezogen auf Gewichtsteile beträgt.

5. Verfahren nach einem oder mehreren der Ansprüche 1 bis 4, **dadurch gekennzeichnet**, die Verarbeitungstemperatur bei den Verfahrensschritten a) und b) 50 bis 200 °C beträgt.

6. Verfahren nach einem oder mehreren der Ansprüche 1 bis 5, **dadurch gekennzeichnet, daß** der Wirkstoff, der ein Salz einer basischen Substanz ist, zur Wirkstoffklasse der Analgetika, Antirheumatika, Psychopharmaka, Antibiotika, Betablocker, Antidiabetika, H1 Antihistaminika, H2 Antihistaminika und/oder Vitamine gehört.

7. Verfahren nach einem oder mehreren der Ansprüche 1 bis 6, **dadurch gekennzeichnet, daß** das Wirkstoff-Salz Verapamil-HCl, Levacetylmethadol hydrochlorid, Oxycodon hydrochlorid, -hydrochlorid-3-Wasser, Tramadol hydrochlorid, Tilidin hydrochlorid, Prometazin embonat; -hydrochlorid, -teoclat, Donepezil hydrochlorid, Nefazodon hydrochlorid, Reboxetin mesilat, Sertralin hydrochlorid, Erythromycin-acistrat, Erythromycin-estolat, Erythromycin-ethylsuccinat, Erythromycin-glucoheptonat, Erythromycin-lactobionat, Erythromycin-propionat, Erythromycin-stearat, Erythromycin-stinoprat, Grepafloxacin hydrochlorid, Ciprofloxacin hydrochlorid-1-Wasser, Levofloxacin hydrochlorid, -lactat, Trovafloxacin mesylat, Nevirapin hydrochlorid, Chlorhexidin acetat, -diacetat-hydrat, -dihydrochlorid, -digluconat, - gluconat, Metronidazol benzoat, Tetracyclin hydrochlorid, -phosphat, Chlortetracyclin hydrochlorid, Oxytetracyclin hydrochlorid, Doxycyclin hyclat, Minocyclin hydrochlorid-hydrat, Neomycin sulfat, Tobramycin sulfat, Clindamycin hydrochlorid, Moxifloxacin hydrochlorid, Indinavir sulfat, Saquinavir mesylat, Nelfinavir mesylat, Amatidin hydrochlorid, Streptomycin sulfat,Amikacin-bis-hydrogensulfat, Paromomycinsulfat, Tobramycinsulfat, Propanolol hydrochlorid, Metoprolol tartrat, -fumarat, Bisoprolol fumarat, Nebivofol hydrochlorid, Betaxolol hydrochlorid, Tertatolol hydrochlorid, Bopindolol malonat, Esmolol hydrochlorid, Oxprenolol hydrochlorid, Metformin hydrochlorid, Pioglitazon hydrochlorid, Rosiglitazon maleat, Diphenhydramin acefyllinat, -citrat,-hydrochlorid, -mesilat, Fexofenadin hydrochlorid, Cimetidin hydrochlorid, Ticlopidin hydrochlorid, Ranitidin hydrochlorid, Roxatidin acetat, Thiamin disulfid, -disulfid-0,-O-dinicotinat, -hydrobromid, -hydrochlorid, -nitrat, Chinidin gluconat; hydrogensulfat-4-Wasser; -lactat, -nitrit, - polygalacturonat, -sulfat, -sulfat-2-Wasser, Amiloprilose, Pseudoephedrin hydrochlorid, Sildenafil-citrat, Granisetron hydrochlorid, Chinin sulfat-2-Wasser; -monohydrochlorid -2-Wasser, Metoclopramid dihydrochtorid-1-Wasser; -hydrochlorid, Pentoxyverin-dihydrogencitrat, -hydrochlorid ist.

8. Verfahren nach Anspruch 7 **dadurch gekennzeichnet, daß** der pH des Granulats oder Pulvers in wässriger Suspension pH 2,3 bis pH 5,0 beträgt.

9. Wirkstoffhaltige Granulate oder Pulver, herstellbar nach einem oder mehreren der Ansprüche 1 bis 8.

10. Verwendung von wirkstoffhaltigen Granulaten oder Pulvern nach Anspruch 9 zur Herstellung von Arzneiformen, Teilen oder von Vorstufen von Arzneiformen.

## Claims

1. Process for the production of active ingredient-containing granules or powders by the steps of
a) melting a mixture of a pharmaceutical active ingredient and of a (meth)-acrylate copolymer which consists of 40 to 75% by weight of free-radically polymerized C₁- to C₄-alkyl esters of acrylic or of methacrylic acid and 25 to 60% by weight of (meth)acrylate monomers having an anionic group in the alkyl radical,
b) extruding the mixture
c) comminuting the extrudate to give granules or powder
**characterized in that**
the processing temperature is 0 to 100°C above the glass transition temperature *T*mg, to be determined without plasticizer addition, according to ISO 11357-2, item 3.3.3, of the (meth)acrylate copolymer and the active ingredient is the salt of a basic substance and **in that** the pH measurable in the powder or granules obtained is pH 7.0 or less than pH 7.0.

2. Process according to Claim 1, **characterized in that** no or less than 0.1% by weight of release agents are added to the mixture.

3. Process according to Claim 1 or 2, **characterized in that** no or less than 0.5% by weight of plasticizers are added to the mixture.

4. Process according to one or more of Claims 1 to 3, **characterized in that** the quantitative ratio of active ingredient to (meth)acrylate copolymer is 10 to 1 to 1 to 10 based on parts by weight.

5. Process according to one or more of Claims 1 to 4, **characterized in that** the processing temperature in process steps a) and b) is 50 to 200°C.

6. Process according to one or more of Claims 1 to 5, **characterized in that** the active ingredient, which is a salt of a basic substance, belongs to the active ingredient class of the analgesics, antirheumatics, psychopharmaceuticals, antibiotics, beta-blockers, antidiabetics, H1 antihistaminics, H2 antihistaminics and/or vitamins.

7. Process according to one or more of Claims 1 to 6, **characterized in that** the active ingredient salt is verapamil HCl, levacetylmethadol hydro-chloride, oxycodone hydrochloride, oxycodone hydrochloride 3-water, tramadol hydrochloride, tilidine hydrochloride, prometazine embonate; prometazine hydrochloride, prometazine teoclate, donepezil hydrochloride, nefazodone hydrochloride, reboxetine mesilate, sertraline hydrochloride, erythromycin acistrate, erythromycin estolate, erythromycin ethylsuccinate, erythromycin glucoheptonate, erythromycin lactobionate, erythromycin propionate, erythromycin stearate, erythromycin stinoprate, grepafloxacin hydrochloride, ciprofloxacin hydrochloride 1-water, levofloxacin hydrochloride, levofloxacin lactate, trovafloxacin mesylate, nevirapine hydrochloride, chlorhexidine acetate, chlorhexidine diacetate hydrate, chlorhexidine dihydrochloride, chlorhexidine digluconate, chlorhexidine gluconate, metro-nidazole benzoate, tetracycline hydrochloride, tetracycline phosphate, chlortetracycline hydro-chloride, oxytetracycline hydrochloride, doxy-cycline hyclate, minocycline hydrochloride hydrate, neomycin sulfate, tobramycin sulfate, clindamycin hydrochloride, moxifloxacin hydro-chloride, indinavir sulfate, saquinavir mesylate, nelfinavir mesylate, amatidine hydrochloride, streptomycin sulfate, amikacin bishydrogensulfate, paromomycin sulfate, tobramycin sulfate, propanolol hydrochloride, metoprolol tartrate, metoprolol fumarate, bisoprolol fumarate, nebivolol hydrochloride, betaxolol hydrochloride, tertatolol hydrochloride, bopindolol malonate, esmolol hydrochloride, oxprenolol hydrochloride, metformin hydrochloride, pioglitazone hydro-chloride, rosiglitazone maleate, diphenhydramine acefyllinate, diphenhydramine citrate, diphen-hydramine hydrochloride, diphenhydramine mesilate, fexofenadine hydrochloride, cimetidine hydro-chloride, ticlopidine hydrochloride, ranitidine hydrochloride, roxatidine acetate, thiamine disulfide, thiamine disulfide O,-O-dinicotinate, thiamine hydrobromide, thiamine hydrochloride, thiamine nitrate, quinidine gluconate; quinidine hydrogen-sulfate 4-water; quinidine lactate, quinidine nitrite, quinidine polygalacturonate, quinidine sulfate, quinidine sulfate 2-water, amiloprilose, pseudoephedrine hydrochloride, sildenafil citrate, granisetrone hydrochloride, quinine sulfate 2-water; quinine monohydrochloride 2-water, meto-clopramide dihydrochloride 1-water; metoclopramide hydrochloride, pentoxyverine dihydrogencitrate, pentoxyverine hydrochloride.

8. Process according to Claim 7 **characterized in that** the pH of the granules or powder in aqueous suspension is pH 2.3 to pH 5.0.

9. Active ingredient-containing granules or powders obtainable according to one or more of Claims 1 to 8.

10. Use of active ingredient-containing granules or powders according to Claim 9 in the manufacture of pharmaceutical forms, parts or of precursors of pharmaceutical forms.

## Revendications

1. Procédé de production de granulés ou de poudres contenant un ingrédient actif, par les étapes :
a) de fusion d'un mélange d'un ingrédient actif pharmaceutique et d'un copolymère de (méth)acrylate qui est constitué de 40 à 75 % en poids d'esters C₁-C₄-alkyliques de l'acide acrylique ou méthacrylique, polymérisés par voie radicalaire, et de 25 à 60 % en poids de monomères de (méth)acrylate renfermant un groupe anionique dans le radical alkyle,
b) d'extrusion du mélange,
c) de concassage de l'extrudat pour donner lieu à un granulé ou une poudre,
**caractérisé :**
**en ce que** la température de mise en oeuvre est de 0 à 100°C au-dessus de la température de transition vitreuse *T*_{mg} du copolymère (méth)acrylate, à déterminer sans l'addition d'agent plastifiant, conformément à ISO 11357-2, article 3.3.3, et l'ingrédient actif est le sel d'une substance basique, et en ce que le pH mesurable pour la poudre ou le granulé obtenu(e) est un pH de 7,0 ou un pH inférieur à 7,0.

2. Procédé selon la revendication 1, **caractérisé en ce que** de 0 à moins de 0, 1 % en poids d'agents de démoulage est ajouté au mélange.

3. Procédé selon la revendication 1 ou 2, **caractérisé en ce que** de 0 à moins de 0,5 % en poids d'agent plastifiant est ajouté au mélange.

4. Procédé selon l'une ou plusieurs des revendications 1 à 3, **caractérisé en ce que** le rapport de quantités entre l'ingrédient actif et le copolymère de (méth)acrylate est de 10:1 à 1:10, par rapport à des parties en poids.

5. Procédé selon l'une ou plusieurs des revendications 1 à 4, **caractérisé en ce que** la température de mise en oeuvre dans les étapes du procédé a) et b) est de 50 à 200°C.

6. Procédé selon l'une ou plusieurs des revendications 1 à 5, **caractérisé en ce que** l'ingrédient actif, qui est un sel d'une substance basique, appartient à la classe d'ingrédients actifs des analgésiques, des anti-rhumatiques, des agents psychopharmaceutiques, des antibiotiques, des bêta-bloqueurs, des antidiabétiques, des antihistaminiques H1, des antihistaminiques H2 et/ou des vitamines.

7. Procédé selon l'une ou plusieurs des revendications 1 à 6, **caractérisé en ce que** le sel d'ingrédient actif est le vérapamil HCl, le chlorhydrate de lévacétylméthadol, le chlorhydrate d'oxycodone, le chlorhydrate-3-eau d'oxycodone, le chlorhydrate de tramadol, le chlorhydrate de tilidine, l'embonate de prométazine, le chlorhydrate de prométazine, le téoclate de prométazine, le chlorhydrate de donépézil, le chlorhydrate de néfazodone, le mésilate de réboxétine, le chlorhydrate de sertraline, l'acistrate d'érythromycine, l'estolate d'érythromycine, l'éthylsuccinate d'érythromycine, le glucoheptonate d'érythromycine, le lactobionate d'érythromycine, le propionate d'érythromycine, le stéarate d'érythromycine, le stinoprate d'érythromycine, le chlorhydrate de grépa-floxacine, le chlorhydrate-1-eau de ciprofloxacine, le chlorhydrate de lévofloxacine, le lactate de lévofloxacine, le mésylate de trovafloxacine, le chlorhydrate de névirapine, l'acétate de chlorhexidine, le diacétate-hydrate de chlorhexidine, le dichlorhydrate de chlorhexidine, le digluconate de chlorhexidine, le gluconate de chlorhexidine, le benzoate de métronidazole, le chlorhydrate de tétracycline, le phosphate de tétracycline, le chlorhydrate de chlortétracycline, le chlorhydrate d'oxy-tétracycline, l'hyclate de doxycycline, le chlorhydrate-hydrate de minocycline, le sulfate de néomycine, le sulfate de tobramycine, le chlorhydrate de clindamycine, le chlorhydrate de moxifloxacine, le sulfate d'indinavir, le mésylate de saquinavir, le mésylate de nelfinavir, le chlorhydrate d'amatidine, le sulfate de streptomycine, le bis-hydrogénosulfate d'amikacine, le sulfate de paromomycine, le sulfate de tobramycine, le chlorhydrate de propanolol, le tartrate de métoprolol, le fumarate de métoprolol, le fumarate de bisoprolol, le chlorhydrate de nébivolol, le chlorhydrate de bétaxolol, le chlorhydrate de tertatolol, le malonate de bopindolol, le chlorhydrate d'esmolol, le chlorhydrate d'oxprénolol, le chlorhydrate de metformine, le chlorhydrate de pioglitazone, le maléate de rosiglitazone, l'acéfyllinate de diphénhydramine, le citrate de diphénhydramine, le chlorhydrate de diphénhydramine, le mésilate de diphénhydramine, le chlorhydrate de fexofénadine, le chlorhydrate de cimétidine, le chlorhydrate de ticlopidine, le chlorhydrate de ranitidine, l'acétate de roxatidine, le disulfure de thiamine, le disulfure-0,-0-dinicotinate de thiamine, le bromhydrate de thiamine, le chlorhydrate de thiamine, le nitrate de thiamine, le gluconate de quinidine, l'hydrogénosulfate-4-eau de quinidine, le lactate de quinidine, le nitrite de quinidine, le polygalacturonate de quinidine, le sulfate de quinidine, le sulfate-2-eau de quinidine, l'amiloprilose, le chlorhydrate de pseudo-éphédrine, le citrate de sildénafil, le chlorhydrate de granisétrone, le sulfate-2-eau de quinine, le monochlorhydrate-2-eau de quinine, le dichlorhydrate-1-eau de métoclopramide, le chlorhydrate de métoclopramide, le dihydro-génocitrate de pentoxyvérine ou le chlorhydrate de pentoxyvérine.

8. Procédé selon la revendication 7, **caractérisé en ce que** le pH du granulé ou de la poudre en suspension aqueuse est un pH de 2,3 à un pH de 5,0.

9. Granulés ou poudres contenant un ingrédient actif pouvant être préparés selon l'une ou plusieurs des revendications 1 à 8.

10. Utilisation de granulés ou de poudres contenant un ingrédient actif selon la revendication 9, pour la production de formes pharmaceutiques, de parties ou de précurseurs de formes pharmaceutiques.
